# EUROPEAN PATENT SPECIFICATION

(11) **EP 4 549 421 B1**
(45) Date of publication and mention of the grant of the patent: **26.08.2026**
(21) Application number: 23206622.5
(22) Date of filing: 30.10.2023
(51) Int. Cl.: C07C 17/10, C07C 19/04, C07C 19/041, C07C 19/043, C07C 19/045, C07C 19/01, C07C 22/04

(54) **METHOD FOR PHOTOCHEMICAL CHLORINATION OF ALKANES**
VERFAHREN ZUR PHOTOCHEMISCHEN CHLORIERUNG VON ALKANEN
PROCÉDÉ DE CHLORATION PHOTOCHIMIQUE D'ALCANES

(43) Date of publication of application: 07.05.2025
(73) Proprietor: Freie Universität Berlin, 14195 Berlin (DE)
(72) Inventor: HASENSTAB-RIEDEL, Sebastian, 14532 Kleinmachnow (DE); KLEOFF, Merlin, 14165 Berlin (DE); VOßNACKER, Patrick, 14165 Berlin (DE); FARIA VIEIRA, André, 12167 Berlin (DE)
(74) Representative: Maikowski & Ninnemann Patentanwälte Partnerschaft mbB

(56) References cited:
- US-A1- 2016 152 533
- US-A1- 2021 087 057

## Description

Chlorinated aliphatics are important basic chemicals in industry, produced on a scale of millions of tonnes per year.

Several chlorinated alkanes are produced commercially, such as methyl chloride, methylene chloride, carbon tetrachloride, and ethyl chloride. These products have a large number of uses. The chlorine derivatives of methane are used as industrial solvents. The principal use of ethyl chloride is as an intermediate in the manufacture of tetraethyllead. All of the chlorinated alkanes can be produced by thermal chlorination of the corresponding alkanes, generally at temperatures above about 250-300 °C. and frequently up to about 600 °C. A thermal chlorination method is for example described in US 2,868,852.

Methods for alkane chlorination at room temperature have also been described. For example, US 11, 578, 021 B1 describes a process for alkane chlorination comprising: (a) providing an aqueous solution comprising dissolved alkanes selected from methane, ethane or combinations thereof: (b) providing an 0.005 to 0.050 M aqueous solution of trichloroisocyanuric acid, wherein the trichloroisocyanuric acid in solution forms cyanuric acid and hypochlorous acid, and (c) contacting the aqueous solution comprising dissolved alkanes with the aqueous solution of trichloroisocyanuric acid, wherein a liquid phase reaction between the dissolved alkanes and the hypochlorous acid forms a gaseous product stream comprising at least one of chloromethane and chloroethane. Trichloroisocyanuric acid itself is obtained by chlorination of cyanuric acid. US2016/152533A1 discloses a method for photochlorination by a photochemical reaction of an aromatic compound with gaseous chlorine so as to prepare a trichloromethyl-substituted benzene, and to a method using bis-(trichloromethyl)-benzene as the trichloromethyl-substituted benzene to prepare by further reaction bis-(chloroformyl)-benzene.

It would be of an advantage to provide alternative synthetic methods for the chlorination of aliphatic hydrocarbons. It would be in particular of an advantage to provide alternative chlorine source that enable chlorination under mild conditions.

This object has been solved by providing a chlorination reaction of alkanes involving or comprising a radical reaction. This may be achieved in a first aspect applying a photochemical reaction and a another, second aspect using a radical starter, like AIBN.

Accordingly, in a first aspect a method for photochemical chlorination of alkanes is provided, wherein the method comprises the following steps:
providing a mixture of at least one polychloride according to general formulae (I)

   [N-R¹ₐR²_{b}R³_{c}R⁴_{d}] [Cl-(Cl₂)ₙ] (I)

   wherein the moities R¹, R², R³ and R⁴ are alkyl, preferably C1-C6 alkyl, or aryl, preferably C6-C10 aryl,
   wherein R¹, R², R³ and R⁴ can be the same or different from each other,
   Wherein a, b, c, d are independently from each other 0, 1, 2 or 3, wherein the sum of a+b+c+d always has to be 4;
   Wherein n > 0, preferably n >= 1, more preferably n = 1-6, even more preferably n = 1, 2, 3, 4, and
at least one alkane according to general formulae (II)

   CR⁵,R ⁶ _{f}R ⁷_{g}R⁸ₕ (II)

   wherein the moities R⁵, R⁶, R⁷ and R⁸ are H, alkyl, aryl, halogen or two of R⁵, R⁶, R⁷ and R⁸ being part of a non-aromatic cyclic ring system,
   wherein R⁵, R⁶, R⁷ and R⁸ can be the same or different from each other,
   wherein at least one of the moities R⁵, R⁶, R⁷ and R⁸ is H,
   wherein e, f, g, h are independently from each other 0, 1, 2 or 3, 4 wherein the sum of e+f+g+h always has to be 4; and
irridiating the mixture at a wavelength in the range between 350 nm and 1000 nm, preferably in the visable range between 400 nm and 800 nm, more preferably between 420 nm and 780 nm, even more preferably between 420 nm and 600 nm.

The present method uses polychlorides according to formulae (I) as chlorination agents that can be activated with visible light, producing chlorine radicals. The chlorine radicals formed are able to chlorinate C(sp3)-H bonds radically.

The polychloride [N-R¹ₐR²_{b}R³_{c}R⁴_{d}] [Cl-(Cl₂)ₙ] is irridiated in the presence of an alkane, and optionally in the presence of a solvent (e.g. 1,2-dichlorobenzene, nitrobenzene, benzene) with visible light. The corresponding chlorinated alkanes and the ionic liquid [N-R¹ₐR²_{b}R³_{c}R⁴_{d}] [Cl-(HCl)ₘ] are formed as by-products. The present method allows for example the conversion of methane to the industrially important, chlorinated compounds CH₃Cl, CH₂Cl₂, CHCl₃ and CCl₄. The product ratio depends on the equivalents of the polychloride [N-R¹ₐR²_{b}R³_{c}R⁴_{d}] [Cl-(Cl₂)ₙ] used and the chlorine loading of the polychloride.

Methyl, methylene and methine carbons of open-chain and cyclic systems can be converted to the corresponding chlorinated aliphatics under the same reaction conditions, as well as chlorination of benzylic systems (aryl-CH₃) to the corresponding chlorinated compounds aryl-CH₂Cl, aryl-CHCl₂ and aryl-CCl₃.

LED chips with an emission maximum at 420 nm were primarily used as the light source, although light sources with other frequency ranges can also be used.. This method eliminates the use of toxic and difficult-to-handle chlorine gas, which is associated with a significant safety hazard.

In an embodiment of the present method, the moities R¹, R², R³ and R⁴ of the polychloride according to general formulae (I) are selected from the following group: methyl, ethyl, n-propyl, isopropyl, n-butyl, isobutyl, and 2-methylpropyl, preferably methyl, ethyl or n-propyl.

It is furthermore preferred, that in the ionic compound of general formula (I) a, b, c = 1, 2 or 3, and d = 0, 1, wherein the sum of a+b+c+d always has to be 4.

The ionic compound of general formula (I) may be selected from [NEt₃Me][Cl(Cl₂)ₙ], [NEt₂Me₂][Cl(Cl₂)ₙ], [NEₜMe₃][Cl(Cl₂)ₙ], [NBuEt₂Me][Cl(Cl₂)ₙ], [NPr₃Me][Cl(Cl₂)ₙ], [NBu₂Me₂][Cl(Cl₂)ₙ], with n being 1-6, preferably 1-4.

In a preferred embodiment, the ionic compound of general formula (I) is selected from [NEt₃Me][Cl(Cl₂)ₙ], [NEt₂Me₂][Cl(Cl₂)ₙ], [NEtMe₃][Cl(Cl₂)ₙ], with n being 1-6, preferably 1-4.

As mentioned, n can be >0 (i.e. any number that is larger 0). For example, if n is 0 < n < 1, then a mixture of [N-R¹ₐR²_{b}R³_{c}R⁴_{d}] [Cl] and [N-R¹ₐR²_{b}R³_{c}R⁴_{d}] [Cl-Cl₂]is present in the storage medium. In such a case, n could be for example 0.8 as in [N-R¹ₐR²_{b}R³_{c}R⁴_{d}] [Cl-(Cl₂)_{0.8}].

The polychlorides of general formula (I) may contain (in the loaded state) at least 0.1 g of Cl₂ per g of ionic compound, preferably at least 0.2 g of Cl₂ per g of ionic compound, more preferably at least 0.3 g of Cl₂ per g of ionic compound, even more preferably at least 0.45 g of Cl₂ per g of ionic compound (at 1 bar, 25°C).

The polychlorides [N-R¹ₐR²_{b}R³_{c}R⁴_{d}] [Cl-(Cl₂)ₙ] of general formulae (I) may be synthesized according to a method as described in WO 2019/215037 A1.

WO 2019/215037 A1 provides a method for separating and storing (loading) chlorine Cl₂ from Cl₂ containing gas, in particular Cl₂ containing process gas, wherein the Cl₂ containing gas is brought into contact with an ionic compound of the structure [NR¹ₐR²_{b}R³_{c}R⁴_{d}] [Cl] to form [N-R¹ₐR²_{b}R³_{c}R⁴_{d}] [Cl-(Cl₂)ₙ] of general formulae (I).

Specifically, WO 2019/215037 A1 describes the use of ionic compounds [NEt₃Me]Cl and [NEt₂Me₂]Cl for reversibly absorbing and storing chlorine from process gas, and which can release the chlorine gas by changing the ambient conditions. Said storage system can be reused after chlorine unloading. When chlorine gas is added to [NEt₃Me]Cl, the corresponding trichloride [NEt₃Me][Cl₃] forms, which at room temperature is an ionic liquid:

[NEt₃Me]Cl + Cl₂ → [NEt₃Me][Cl-(Cl)₂]

Since trichloride [NEt₃Me][Cl₃] is an ionic liquid with a low chlorine vapor pressure, it can be handled and transported much more safely than pressurized chlorine.

In the course of the chlorination reaction, at least one polychloride according to general formulae (I) is converted to at least one compound according to general formulae (III)

[N-R¹ₐR²_{b}R³_{c}R⁴_{d}] [Cl-(HCl)ₘ]

wherein the moities R¹, R², R³ and R⁴ and the variables a, b, c, d are the same as previously described in at least one of the preceding claims, and wherein m > 0, preferably m = 1-6, more preferably m = 1, 2, 3, 4. Thus, m can be >0 (i.e. any number that is larger 0). For example, if m is 0 < m < 1, then a mixture of [N-R¹ₐR²_{b}R³_{c}R⁴_{d}] [CI] and [N-R¹ₐR²_{b}R³_{c}R⁴_{d}] [Cl-HCl] is obtained

In an embodiment of the present method the moities R⁵, R⁶, R⁷ and R⁸ of the at least one alkane according to general formulae (II) are
- H, and/or
- C1-C10 alkyl, preferably C1-C6 alkyl, more preferably C1-C4,
- C6-C10 aryl, preferably C6-C8 aryl, more preferably C6 aryl,
- Halogen selected from Br, Cl or F, in particular Cl, and/or
- two of R⁵, R⁶, R⁷ and R⁸ being part of a C5- C10 non-aromatic cyclic ring system, preferably a C5-C8 non-aromatic cyclic ring system, more preferably a C5-C7 non-aromatic cyclic ring system.

In particualr, the moities R⁵, R⁶, R⁷ and R⁸ of at least one alkane according to general formulae (II) are H, methyl, ethyl, n-propyl, iso-propyl, n-butyl, iso-butyl and sec-butyl, in particular preferably H, methyl, ethyl, n-propyl, n-butyl, and phenyl. Furthrmore, two of the moities R⁵, R⁶, R⁷ and R⁸ may form a cyclohexane ring. It is imperative that at least one of the moities R⁵, R⁶, R⁷ and R⁸ has to be H.

Thus, different alkanes according to general formulae (II) are suitable for chlorination that can be grouped according to different substituion pattern.

If all of R⁵, R⁶, R⁷ and R⁸ are H, the alkane of general formulae (II) is Methane CH₄.

If three of R⁵, R⁶, R⁷, R⁸ are H and only one of R⁵, R⁶, R⁷, R⁸ is an alkyl or aryl, then the alkane of general formulae (II) is an equivalent of a methyl group.

An embodiment may be R⁵-CH₃ wherein R⁶, R⁷, R⁸ are H and R⁵ is one of methyl, ethyl, n-propyl, iso-propyl, n-butyl, iso-butyl and sec-butyl, in particular preferably methyl, ethyl, n-propyl, n-butyl, and phenyl. Specific examples of this group are ethane or toluene.

If two of R⁵, R⁶, R⁷, R⁸ are H and two of R⁵, R⁶, R⁷, R⁸ are an alkyl or aryl, then the alkane of general formulae (II) is an equivalent of a methylene group.

An embodiment may be R⁵-CH₂-R⁶ wherein R⁷, R⁸ are H and R⁵, R⁶ are each or both one of methyl, ethyl, n-propyl, iso-propyl, n-butyl, iso-butyl and sec-butyl, in particular preferably methyl, ethyl, n-propyl, n-butyl, and phenyl. A specific example may be propane.

Furthermore, R⁵, R⁶ may also form or be part of a C5-C7 non-aromatic cyclic ring system, such as an C6 ring; in this case the alkane would be cyclohexane.

If only one of R⁵, R⁶, R⁷, R⁸ is H and three of R⁵, R⁶, R⁷, R⁸ are an alkyl or aryl, then the alkane of general formulae (II) is an equivalent of a methin group.

An embodiment may be CHR⁵R⁶R⁷ wherein R⁸ is H and with three of R⁵, R⁶, R⁷ are each methyl, ethyl, n-propyl, iso-propyl, n-butyl, iso-butyl and sec-butyl, in particular preferably methyl, ethyl, n-propyl, n-butyl, and phenyl. A specific example of this group is iso-butane, wherein each of R⁵R⁶R⁷ is methyl.

Furthermore, R⁵, R⁶ may also form or be part of a C5-C7 non-aromatic cyclic ring system, such as an C6 ring, in this case the alkane would be cyclohexane substituted with R⁷.

It is to be understood that any of the moieties mentioned above can be non-substituted or further substituted.

Here the term "substituted", in particular in connection to alkyl, cycloalkyl, aryl relates to the substitution of one or more atoms, usually H-atoms, by one or more of the following substituents: C₁-C₁₅ alkyl, C₃-C₁₀-cycloalkyl, aryl, heteroaryl, naphthyl, heterocyclic ring, imidazolyl, indolyl, pyrrolidinyl, C₁-C₁₂-alkoxy, C₁-C₁₂-acyl, C₁-C₁₂-acyloxy, carboxy, ester, , C₁-C₁₂-(per)fluoroalkyl and C₁-C₁₀-alkylsulfonyl.

It is in preferred if any of the moieties mentioned above can be non-substituted or substituted with C₁-C₁₅ alkyl, C₃-C₁₀-cycloalkyl, aryl, heteroaryl, naphthyl, C₁-C₁₂-alkoxy.

It is in particular preferred if any of the moieties mentioned above can be non-substituted or substituted with hydroxy, C₁-C₁₀ alkyl, such as methyl, ethyl or propyl, C₃-C₁₀-cycloalkyl, C₁-C₁₂-alkoxy.

The substituted groups can be once or twice substituted with same or different substituents.

In the course of the chlorination reaction, at least one alkane according to general formulae (II) is converted to at least one chlorinated alkane according to general formulae (IV)

(R⁵ₑ,R⁶_{f}R⁷_{g}R⁸ₕ)CClₓ,

Wherein the moities R⁵, R⁶, R⁷ and R⁸ are the same as previously described, x is 1, 2, 3, or 4, wherein e, f, g, h are independently from each other 0, 1, 2 or 3, wherein the sum of e+f+g+h always has to be 4-x (i.e. 4 minus the number of chlorine atoms). Thus, the alkane of general formulae (II) may be chlorinated once or multiple times, preferably one, twice, three or four times.

For example, methane CH₄ may be chlorinated to CH₃Cl, CH₂Cₗ₂, CHCl₃ and/or CCl₄.

A methyl group according to R⁵-CH₃ may be chlorinated to R⁵-CH₂Cl, R⁵-CHCl₂ and/or R⁵-CCl₃. An example is the chlorination of toluene C₆H₅CH₃ to C₆H₅CH₂Cl.

It is to be understood that the chlorination also may occur at the moiety R⁵ providing another chlorination pattern. For example, in case R⁵ is an alkyl moiety (e.g. a methyl moiety) chlorination may also occur at R⁵. An example is ethane C₂H₆ that may undergo chlorination at both C-atoms providing CH₃-CH₂Cl, ClCH₂CH₂Cl and/or Cl₂CH-CH₃.

A methylene group R⁵-CH₂-R⁶ may be chlorinated to R⁵-CHCl-R⁶ and /or R⁵-CCl₂-R⁶.

Also in this case, chlorination also may occur at the moiety R⁵ and/or R⁶ providing another chlorination pattern. For example, in case R⁵ and R⁶ are an alkyl moiety (e.g. a methyl moiety) chlorination may also occur at R⁵ and/or R⁶. An example is propane C₃H₈ that may undergo chlorination at different C-atoms providing CICH₂-CH₂-CH₃, CH₃-CHCl-CH₃, CH₃-CHCl₂-CH₃ and/or ClCH₂-CHCl-CH₃,

A methine group CHR⁵R⁶R⁷ may be chlorinated to CR⁵R⁶R⁷Cl.

Also in this case, chlorination also may occur at any of the moieties R⁵, R⁶ and/or R⁷ providing another chlorination pattern. For example, in case R⁵, R⁶ and R⁷ are an alkyl moiety (e.g. a methyl moiety) chlorination may also occur at R⁵, R⁶ and/or R⁷. An example is isobutane iso-C₄H₁₀ that may undergo chlorination at different C-atoms providing ClCH₂-CH(CH₃)₂, CH₃-CCl(CH₃)₂ and/or ClCH₂-CCl(CH₃)₂

If moieties R⁵, R⁶ are part of a C5-C7 non-aromatic cyclic ring system, such as an C6 ring, then chlorination may not only occur at one methylene or methin group but at several different positions of the cyclic ring system.

In another embodiment of the present method 0.2- 3.0 equiv, preferably 0.5-2.0 equiv, of the at least one polychloride according to general formulae (I) and 1.0 - 4.0 equiv, preferably 2.0-3.0 equiv, of the at least one at least one alkane according to general formulae (II) are provided in the reaction mixture.

In still a further embodiment the reaction is carried out in an organic solvent selected from the group of benzene, nitrobenzene, 1,2-dichlorobenzene (oDCB), fluorinated benzene, trifluorotoluene. In general, any electron deficient aromatic solvent would be suitable that is inert to chlorination and has no functional, reactive substituents

As previously mentioned, the wavelength used for irradiation may be in the range between 350 nm and 1000 nm, preferably in the visable range between 400 nm and 800 nm, morepreferably between 420 nm and 780 nm, even more preferably between 420 nm and 600; such as 365 nm or 420 nm. A a suitable light source may be a LED light emitting light at a wavelength between 420 nm and 600 nm, preferably a blue LED light emitting light at a wavelength between 420 nm and 470 nm. However, other irradiation sources may also be suitable.

As mentioned above, the object of the invention may also be solved by using a radical starter, such as AIBN.

Thus, according to a second aspect of the invention a method for radical chlorination of alkanes is provided, wherein the method comprises the steps of providing a mixture of
at least one polychloride according to general formulae (I)

   [N-R¹ₐR²_{b}R³_{c}R⁴_{d}] [Cl-(Cl₂)ₙ]

   wherein the moities R¹, R², R³ and R⁴ are alkyl, preferably C1-C6 alkyl, or aryl, preferably C6-C10 aryl,
   wherein R¹, R², R³ and R⁴ can be the same or different from each other,
   Wherein a, b, c, d are independently from each other 0, 1, 2 or 3, wherein the sum of a+b+c+d always has to be 4;
   Wherein n >0, preferably n >= 1, more preferably n = 1-6, even more preferably n = 1, 2, 3, 4, and
at least one alkane according to general formulae (II)

   CR⁵ₑR⁶_{f}R⁷_{g}R⁸ₕ

   wherein the moities R⁵, R⁶, R⁷ and R⁸ are H, alkyl, aryl, halogen or two of R⁵, R⁶, R⁷ and R⁸ being part of a non-aromatic cyclic ring system,
   wherein R⁵, R⁶, R⁷ and R⁸ can be the same or different from each other,
   wherein at least one of the moities R⁵, R⁶, R⁷ and R⁸ is H,
   wherein e, f, g, h are independently from each other 0, 1, 2 or 3, 4 wherein the sum of e+f+g+h always has to be 4; and
adding at least one radical starter to the mixtures and heating the mixture to a temperature between 80°C and 120°C, preferably between 90°C and 110°C, more preferably 100°C for 15 to 60 min, preferably 20 to 40 min, more preferably 25 to 35 min.

For further definition of compounds (I), (II) and their respective reaction products the definitions as previously provided for the first method aspect also apply to this second method aspect.

A suitable radical starter may be Azobis(isobutyronitril) (AIBN). Other suitable radical starters may include peroxides, such as dibenzoylperoxide (DBPO).

In an embodiment, the radical starter may be added in an amount between 0.5 and 1.5 mol%, preferably between 0.8 and 1.2 mol%, such as 1 mol%.

In a preferred embodiment, 1.0-mol% of AIBN was added to the reaction mixture as a radical starter and the reaction mixture was warmed to 100 °C for 30 min.

The invention is explained in more detail by means of the following figures and examples. It shows:

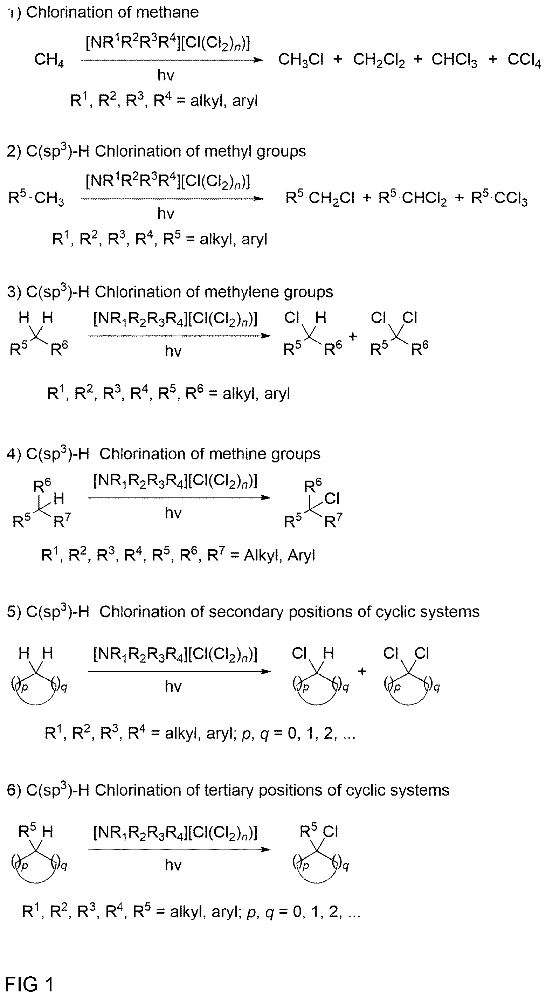

- Figure 1: an overview of different chlorination reactions carried out according to the method of the invention;
- Figure 2: ¹H-NMR Spectrum (400 MHz, CD₃CN, 20 °C) of the reaction products of the photochlorination of CH₄ with [NEt₃Me][Cl₃];
- Figure 3: ¹³C-NMR Spectrum (100 MHz, CD₃CN, 20 °C) of the reaction products of the photochlorination of CH₄ with [NEt₃Me][Cl₃];
- Figure 4: IR-Spectrum of the reaction products of the photochlorination of CH₄ with [NEt₃Me][Cl₃].
- Figure 5: ¹H-NMR spectrum (400 MHz, CDCl₃, 20 °C) of benzyl chloride; and

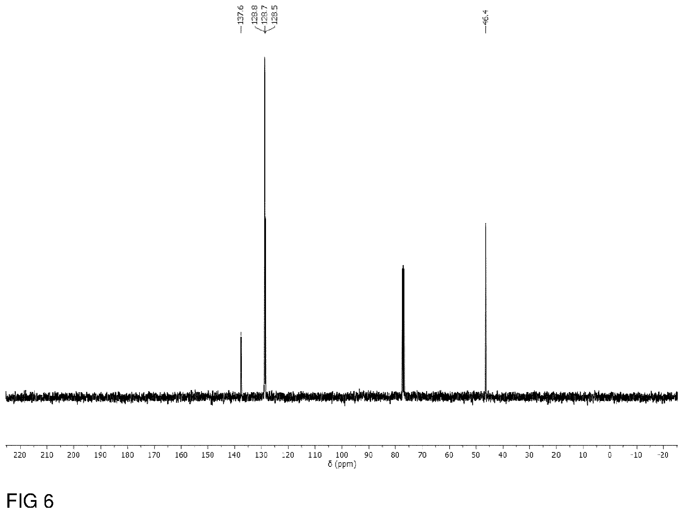

- Figure 6: ¹³C NMR Spectrum (100 MHz, CDCl₃, 20 °C) of benzyl chloride.

### Example 1: Chlorination of Alkanes

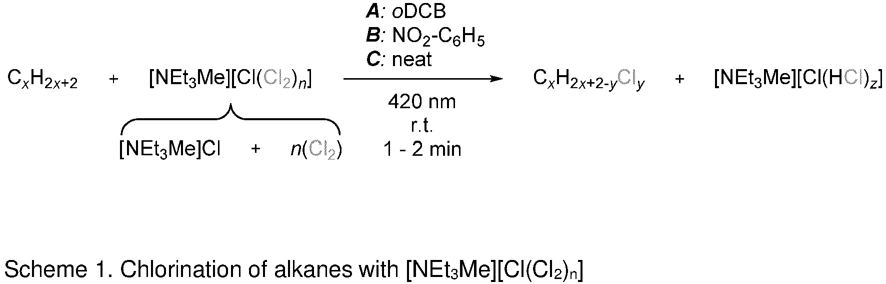

### General Procedure A:

Dry triethyl methyl ammonium chloride [NEt₃Me]Cl (371 mg, 2.45 mmol, 0.35 equiv.) was loaded into a 500-ml Rettberg Schlenk flask and suspended in 1.6 ml oDCB. The suspension was degassed and Cl₂ was introduced, until the system retained a pressure of 200 mbar (493 mg, 6.95 mmol, 1.0 equiv.) yielding in the formation of [NEt₃Me][Cl(Cl₂)*ₙ*] (*n =* 1-3). C*ₓ*H_{2*x*+2} (ca. 16 mmol, 2.3 equiv.) (*x =* 1-4) was added to the reaction vessel. Under vigorous stirring, the reaction mixture was irradiated for 1-2 min with a blue (420 nm) LED light source. The progression of the reaction could be observed by the color loss of the ionic liquid. The reaction products were purified by distillation using two in-series connected cooling traps, the former one at -40 °C (for trapping *o*DCB) and the latter one at -140 °C (C₁ & C₂ entities) or -90°C (C₃ and C₄ entities). The reaction products were characterized by gas phase IR and ¹H-NMR spectroscopy to determine the ratios between the different chlorinated products.

### General Procedure B:

Identical to *Procedure A,* except the use of nitro benzene (NO₂-C₆H₅) instead of *o*DCB.

### General Procedure C:

Dry triethyl methyl ammonium chloride [NEt₃Me]Cl (371 mg, 2.45 mmol, 0.35 equiv.) in a 500-ml Rettberg Schlenk flask was evacuated and Cl₂ was introduced, until the system retained a pressure of 500 mbar (493 mg, 10.3 mmol, 1.0 equiv.). CₓH₂ₓ₊₂ (23.7 mmol, 2.3 equiv.) was condensed into the reaction vessel. Under vigorous stirring, the reaction mixture was irradiated for 1-2 min with a blue (420 nm) LED light source. The subsequent workup and yield analysis is analog to *General Procedure A.*

### General Procedure D:

Identical to *General Procedure A* but instead of irritation with blue light 1.0-mol% of AIBN was added to the reaction mixture as a radical starter and the reaction mixture was warmed to 100 °C for 30 min.

### Example 2: Photochlorination of CH₄

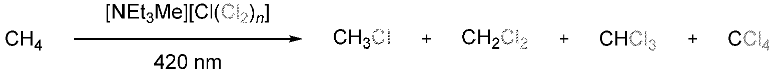

**Table 1. Overview and Summary of photochlorination of CH₄ with [NEt₃Me][Cl(Cl₂)ₙ].**

| Entry | Solvent | Conditions | | | Yield [%] | | | | |
|---|---|---|---|---|---|---|---|---|---|
| | | Temp. | Irradiation Time | Ratio Cl₂:CH₄ | CH₃Cl | CH₂Cl₂ | CHCl₃ | CCl₄ | Absolut Yield |
| 1 | NO₂-C₆H₅ | r.t. | 1 min | 1 : 2.2 | 54 | 42 | 4 | n.a. | 89 |
| 2 | oDCB | r.t. | 1.5-2 min | 1 : 2.3 | 50 | 45 | 5 | n.a. | 74 |
| 3 | neat | r.t. | 1.5 min | 1 : 2.4 | 59 | 35 | 6 | n.a. | 47 |
| 4^{[a]} | oDCB | 40 °C | 1 h | 1 : 2.2 | 0 | 0 | 0 | 0 | 0 |

| | | | | | | | | | |
|---|---|---|---|---|---|---|---|---|---|
| [a] With exclusion of light | | | | | | | | | |

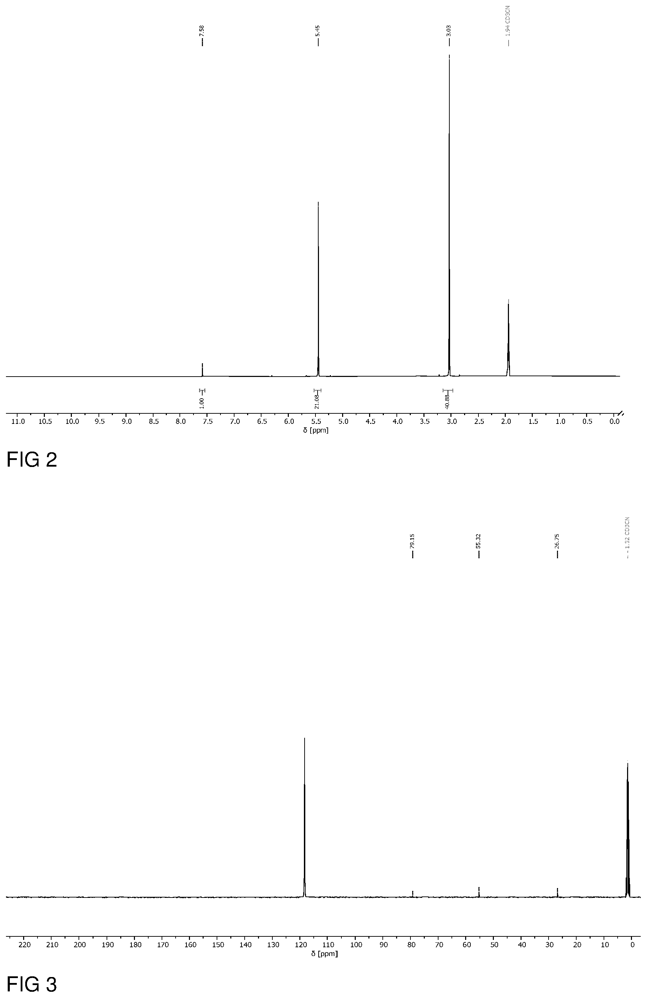

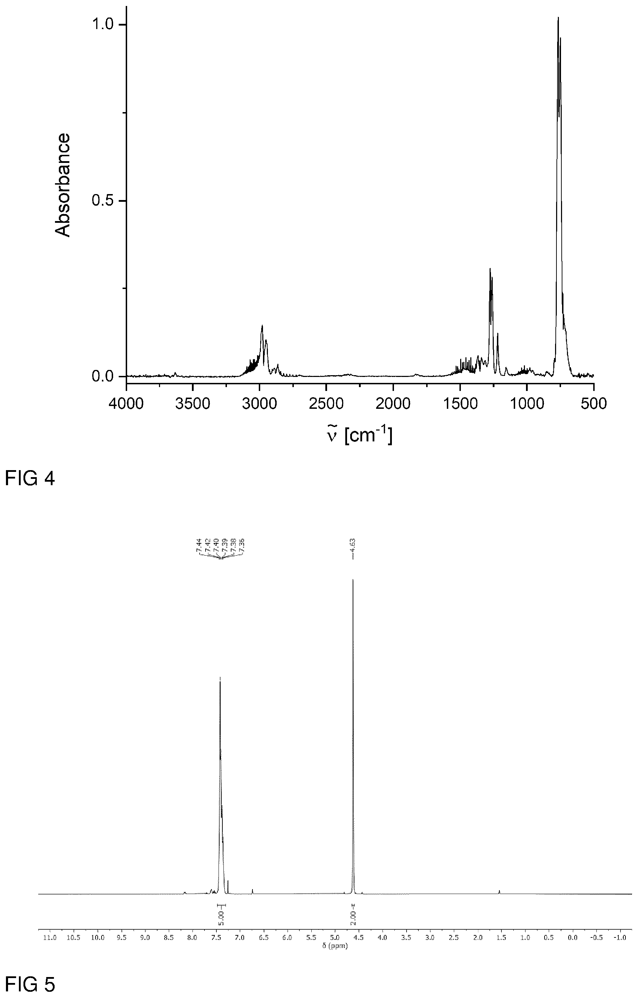

**Characterization:** ¹H NMR (Figure 2), ¹³C NMR (Figure 3), Gas phase IR (Figure 4)
**¹H NMR** (400 MHz, CD₃CN, 295 K): δ [ppm] = 7.58 (*s*, CHCl₃), 5.45 (*s*, CH₂Cl₂), 3.03 (*s*, CH₃Cl). **¹³C NMR** (101 MHz, CD₃CN, 295 K): δ [ppm] = 55.3, 26.8.

### Example 3: Photochlorination of C₂H₆

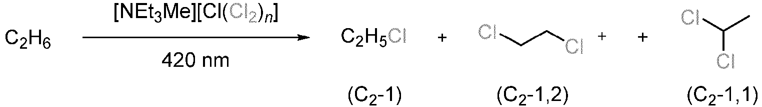

**Table 2. Overview and Summary of photochlorination of C₂H₆ with [NEt₃Me][Cl(Cl₂)ₙ].**

| Entry | Solvent | Conditions | | | Yield [%] | | | |
|---|---|---|---|---|---|---|---|---|
| | | Temp. | Irradiation Time | Ratio Cl₂:C₂H₆ | C₂-1 | C₂-1,2 | C₂-1,1 | Absolut Yield |
| 1 | oDCB | r.t. | 1 min | 1 : 2.2 | 93 | 3 | 4 | 79 |
| 2 | neat | r.t. | 1.5 min | 1 : 1.8 | 90 | 3 | 7 | 81 |
| 3^{[a]} | oDCB | 100 °C | 30 min | 1 : 2.2 | 90 | 3 | 7 | 75 |

| | | | | | | | | |
|---|---|---|---|---|---|---|---|---|
| [a] With exclusion of light and in presence of 1.0-mol% of AIBN | | | | | | | | |

### Example 4: Photochlorination of C₃H₈

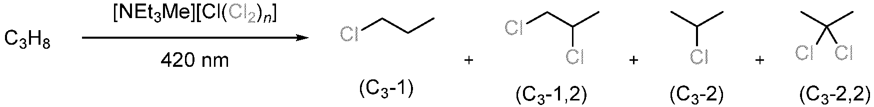

**Table 3. Overview and Summary of photochlorination of C₃H₈ with [NEt₃Me][Cl(Cl₂)ₙ].**

| Entry | Solvent | Conditions | | | Yield [%] | | | | |
|---|---|---|---|---|---|---|---|---|---|
| | | Temp. | Irradiation Time | Ratio Cl₂:C₂H₆ | C₃-1 | C₃-1,2 | C₃-2 | C₃-2,2 | Absolut Yield |
| 1 | neat | r.t. | 1.5 min | 1:2.3 | 48 | 3 | 45 | 3 | 70% |

### Example 5: Photochlorination of iso-C₄H₁₀

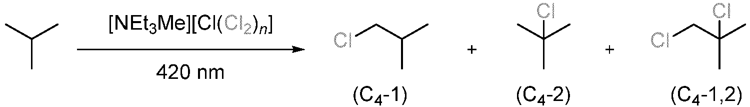

**Table 4. Overview and Summary of photochlorination of C₄H₁₀ with [NEt₃Me][Cl(Cl₂)ₙ].**

| Entry | Solvent | Conditions | | Yield [%] | | | | |
|---|---|---|---|---|---|---|---|---|
| | | Temp. | Irradiation Time | Ratio Cl₂:C₂H₆ | C₄-1 | C₄-2 | C₄-1,2 | Absolut Yield |
| 1 | neat | r.t. | 1 min | 1:2.0 | 57 | 38 | 5 | 66 |

### Example 6: Photochlorination of toluene

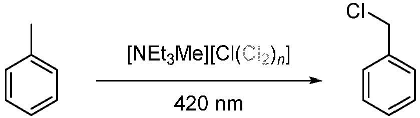

Toluene (1.2 mol, 2.5 equiv., 125 mL) was loaded into a flask. The flask was irradiated with a blue (420 nm) LED light source and [NEt₃Me][Cl(Cl₂)_{1.7}] (77 g, 0.48 mol Cl₂, 1 equiv.) was slowly added yielding a two phase system. Irradiation was continued until the solution decolorized (ca. 1 h). Subsequently, the phases were separated, the organic phase was washed 2x with distilled water (100 mL) and the ionic phase was washed 2x with toluene (50 mL). The combined organic phases were dried over sodium sulfate. Fractional distillation at 100 mbar in static vacuum yielded pure benzyl chloride (0.29 mol, 62%) as a colorless liquid.

¹H-NMR (Figure 4) (400 MHz, CDCl₃) δ = 7.44-7.36 (m, 5H), 4.63 (s, 2H) ppm.
¹³C-NMR (Figure 5) (100 MHz, CDCl₃) δ = 137.6, 128.8, 128.7, 128.5, 46.4 ppm

## Claims

1. Method for photochemical chlorination of alkanes comprising the steps of providing a mixture of
at least one polychloride according to general formulae (I)
[N-R¹ₐR²_{b}R³_{c}R⁴_{d}] [Cl-(Cl₂)ₙ]
wherein the moities R¹, R², R³ and R⁴ are alkyl, preferably C1-C6 alkyl, or aryl, preferably C6-C10 aryl,
wherein R¹, R², R³ and R⁴ can be the same or different from each other,
wherein a, b, c, d are independently from each other 0, 1, 2 or 3, wherein the sum of a+b+c+d always has to be 4;
wherein n >0, preferably n >= 1, more preferably n = 1-6, even more preferably n = 1, 2, 3, 4, and
at least one alkane according to general formulae (II)
CR⁵ₑR⁶_{f}R⁷_{g}R⁸ₕ
wherein the moities R⁵, R⁶, R⁷ and R⁸ are H, alkyl, aryl, halogen or two of R⁵, R⁶, R⁷ and R⁸ being part of a non-aromatic cyclic ring system,
wherein R⁵, R⁶, R⁷ and R⁸ can be the same or different from each other,
wherein at least one of the moities R⁵, R⁶, R⁷ and R⁸ is H,
wherein e, f, g, h are independently from each other 0, 1, 2 or 3, 4 wherein the sum of e+f+g+h always has to be 4; and
irridiating the mixture at a wavelength in the range between 350 nm and 1000 nm, preferably in the visable range between 400 nm and 800 nm, preferably between 420 nm and 780 nm, more preferably between 420 nm and 600 nm.

2. Method according to claim 1, **characterized in that** the moities R¹, R², R³ and R⁴ of the polychloride according to general formulae (I) are selected from the following group: methyl, ethyl, n-propyl, isopropyl, n-butyl, isobutyl, and 2-methylpropyl, preferably methyl, ethyl or n-propyl.

3. Method according to one of the preceding claims, **characterized in that** in the ionic compound of general formula (I) a, b, c = 1, 2 or 3, and d = 0, 1, wherein the sum of a+b+c+d always has to be 4.

4. Method according to one of the preceding claims, **characterized in that** the ionic compound of general formula (I) is selected from [NEt₃Me][Cl(Cl₂)ₙ], [NEt₂Me₂][Cl(Cl₂)ₙ], [NEtMe₃][Cl(Cl₂)ₙ], [NBuEt₂Me][Cl(Cl₂)ₙ], [NPr₃Me][Cl(Cl₂)ₙ], [NBu₂Me₂][Cl(Cl₂)ₙ], with n being 1-6, preferably 1-4.

5. Method according to one of the preceding claims, **characterized in that** the ionic compound of general formula (I) is selected from [NEt₃Me][Cl(Cl₂)ₙ], [NEt₂Me₂][Cl(Cl₂)ₙ], [NEtMe₃][Cl(Cl₂)_{n]}, with n being 1-6, preferably 1-4.

6. Method according to one of the preceding claims, **characterized in that** the ionic compound of general formula (I) contains (in the loaded state) at least 0.1 g of Cl₂ per g of ionic compound, preferably at least 0.2 g of Cl₂ per g of ionic compound, more preferably at least 0.3 g of Cl₂ per g of ionic compound, even more preferably at least 0.45 g of Cl₂ per g of ionic compound.

7. Method according to one of the preceding claims, **characterized in that** the moities R⁵, R⁶, R⁷ and R⁸ of the at least one alkane according to general formulae (II) are
- H, and/or
- C1-C10 alkyl, preferably C1-C6 alkyl, more preferably C1-C4,
- C6-C10 aryl, preferably C6-C8 aryl, more preferably C6 aryl,
- halogen selected from Br, Cl or F, in particular Cl, and/or
- two of R⁵, R⁶, R⁷ and R⁸ being part of a C5- C10 non-aromatic cyclic ring system, preferably a C5-C8 non-aromatic cyclic ring system, more preferably a C5-C7 non-aromatic cyclic ring system.

8. Method according to one of the preceding claims, **characterized in that** the moities R⁵, R⁶, R⁷ and R⁸ of the at least one alkane according to general formulae (II) are H, methyl, ethyl, n-propyl, iso-propyl, n-butyl, iso-butyl and sec-butyl, in particular preferably H, methyl, ethyl, n-propyl, n-butyl, phenyl.

9. Method according to one of the preceding claims, **characterized in that** two of the moities R⁵, R⁶, R⁷ and R⁸ form a cyclohexane ring.

10. Method according to one of the preceding claims, characterized that 0.2- 3.0 equiv, preferably 0.3 - 2.0 equiv, of at least one polychloride according to general formulae (I) and 1.0 - 4.0 equiv, preferably 2.0-3.0 equiv, of at least one alkane according to general formulae (II) are provided in the reaction mixture.

11. Method according to one of the preceding claims, **characterized in that** the reaction is carried out in an organic solvent selected from the group of benzene, nitrobenzene, 1,2-dichlorobenzene (*o*DCB), fluorinated benzene, trifluorotoluene.

12. Method according to one of the preceding claims, **characterized in that** the light source is a LED light emitting light at a wavelength between 420 nm and 600 nm, preferably a blue LED light emitting light at a wavelength between 420 nm and 470 nm.

13. Method for radical chlorination of alkanes comprising the steps of providing a mixture of
at least one polychloride according to general formulae (I)
[N-R¹ₐR²_{b}R³_{c}R⁴_{d}] [Cl-(Cl₂)ₙ]
wherein the moities R¹, R², R³ and R⁴ are alkyl, preferably C1-C6 alkyl, or aryl, preferably C6-C10 aryl,
wherein R¹, R², R³ and R⁴ can be the same or different from each other,
wherein a, b, c, d are independently from each other 0, 1, 2 or 3, wherein the sum of a+b+c+d always has to be 4;
wherein n >0, preferably n >= 1, more preferably n = 1-6, even more preferably n = 1, 2, 3, 4, and
at least one alkane according to general formulae (II)
CR⁵ₑR⁶_{f}R⁷_{g}R⁸ₕ
wherein the moities R⁵, R⁶, R⁷ and R⁸ are H, alkyl, aryl, halogen or two of R⁵, R⁶, R⁷ and R⁸ being part of a non-aromatic cyclic ring system,
wherein R⁵, R⁶, R⁷ and R⁸ can be the same or different from each other,
wherein at least one of the moities R⁵, R⁶, R⁷ and R⁸ is H,
wherein e, f, g, h are independently from each other 0, 1, 2 or 3, 4 wherein the sum of e+f+g+h always has to be 4; and
adding at least one radical starter to the mixtures and heating the mixture to a temperature between 80 and 120°C, preferably between 90 and 110°C, more preferably 100°C for 15 to 60 min, preferably 20 to 40 min, more preferably 25 to 35 min.

14. Method according to one of the preceding claims, **characterized in that** at least one polychloride according to general formulae (I) is converted to at least one compound according to general formulae (III)
[N-R¹ₐR²_{b}R³_{c}R⁴_{d}] [Cl-(HCl)ₘ]
wherein the moities R¹, R², R³ and R⁴ and the variables a, b, c, d are the same as previously described in at least one of the preceding claims, and
wherein m>0, preferably m >= 1, more preferably m = 1-6, even more preferably m = 1, 2, 3, 4.

15. Method according to one of the preceding claims, **characterized in that** at least one alkane according to general formulae (II) is converted to at least one chlorinated alkane according to general formulae (IV)
(R⁵ₑR⁶_{f}R⁷_{g}R⁸ₕ)CClₓ
wherein at least one of the moities R⁵, R⁶, R⁷ and R⁸ is the same as previously described in at least one of the preceding claims,
x is 1, 2,3, or 4,
wherein R⁵, R⁶, R⁷ and R⁸ can be the same or different from each other, and
wherein e, f, g, h are independently from each other 0, 1, 2 or 3, wherein the sum of e+f+g+h always has to be 4-x. (i.e. 4 minus the number of chlorine atoms).

## Patentansprüche

1. Verfahren zur photochemischen Chlorierung von Alkanen, umfassend die Schritte des Bereitstellens einer Mischung aus
mindestens einem Polychlorid gemäß der allgemeinen Formel (I)
[N-R¹ₐ R²_{b} R³_{c} R⁴_{d} ] [Cl-(Cl₂ )ₙ]
wobei die Reste R¹ , R², R³ und R⁴ Alkyl, vorzugsweise C1-C6-Alkyl, oder Aryl, vorzugsweise C6-C10-Aryl, sind,
wobei R¹, R², R³ und R⁴ gleich oder voneinander verschieden sein können,
wobei a, b, c, d unabhängig voneinander 0, 1, 2 oder 3 sind, wobei die Summe aus a+b+c+d stets 4 betragen muss;
wobei n > 0, vorzugsweise n >= 1, noch bevorzugter n = 1-6, am meisten bevorzugt n = 1, 2, 3, 4 ist, und
mindestens einem Alkan gemäß der allgemeinen Formel (II)
CR⁵ₑ R⁶_{f} R⁷_{g} R⁸ₕ
wobei die Reste R⁵ , R⁶, R⁷ und R⁸ H, Alkyl, Aryl oder Halogen sind oder zwei der Reste R⁵ , R⁶ , R⁷ und R⁸ Teil eines nicht-aromatischen cyclischen Ringsystems sind,
wobei R⁵, R⁶, R⁷ und R⁸ gleich oder voneinander verschieden sein können,
wobei mindestens einer der Reste R⁵, R⁶, R⁷und R⁸ H ist,
wobei e, f, g, h unabhängig voneinander 0, 1, 2 oder 3 sind, wobei die Summe aus e + f + g + h stets 4 betragen muss; und
Bestrahlung der Mischung bei einer Wellenlänge im Bereich zwischen 350 nm und 1000 nm, vorzugsweise im sichtbaren Bereich zwischen 400 nm und 800 nm, vorzugsweise zwischen 420 nm und 780 nm, noch bevorzugter zwischen 420 nm und 600 nm.

2. Verfahren nach Anspruch 1, **dadurch gekennzeichnet, dass** die Reste R¹, R², R³ und R⁴ des Polychlorids gemäß der allgemeinen Formel (I) aus der folgenden Gruppe ausgewählt sind: Methyl, Ethyl, n-Propyl, Isopropyl, n-Butyl, Isobutyl und 2-Methylpropyl, vorzugsweise Methyl, Ethyl oder n-Propyl.

3. Verfahren nach einem der vorstehenden Ansprüche, **dadurch gekennzeichnet, dass** in der ionischen Verbindung der allgemeinen Formel (I) a, b, c = 1, 2 oder 3 und d = 0, 1 sind, wobei die Summe aus a+b+c+d stets 4 betragen muss.

4. Verfahren nach einem der vorstehenden Ansprüche, **dadurch gekennzeichnet, dass** die ionische Verbindung der allgemeinen Formel (I) ausgewählt ist aus [NEt₃ Me][Cl(Cl₂)ₙ], [NEt₂Me₂][Cl(Cl₂)ₙ], [NEtMe₃][Cl(Cl₂)ₙ], [NBuEt₂Me][Cl(Cl₂)ₙ], [NPr₃Me][Cl(Cl₂)ₙ], [NBu₂Me₂][Cl(Cl₂)ₙ], wobei n 1-6, vorzugsweise 1-4 beträgt.

5. Verfahren nach einem der vorstehenden Ansprüche, **dadurch gekennzeichnet, dass** die ionische Verbindung der allgemeinen Formel (I) ausgewählt ist aus [NEt₃ Me][Cl(Cl₂)ₙ], [NEt₂ Me₂][Cl(Cl₂)ₙ], [NEtMe₃][Cl(Cl₂)ₙ], wobei n 1 bis 6, vorzugsweise 1 bis 4 beträgt.

6. Verfahren nach einem der vorstehenden Ansprüche, **dadurch gekennzeichnet, dass** die ionische Verbindung der allgemeinen Formel (I) (im beladenen Zustand) mindestens 0,1 g Cl₂ pro g ionischer Verbindung, vorzugsweise mindestens 0,2 g Cl₂ pro g ionischer Verbindung, noch bevorzugter mindestens 0,3 g Cl₂ pro g ionischer Verbindung, noch bevorzugter mindestens 0,45 g Cl₂ pro g ionischer Verbindung.

7. Verfahren nach einem der vorstehenden Ansprüche, **dadurch gekennzeichnet, dass** die Reste R⁵, R⁶, R⁷und R⁸ des mindestens einen Alkans gemäß der allgemeinen Formel (II)
- H und/oder
- C1-C10-Alkyl, vorzugsweise C1-C6-Alkyl, noch bevorzugter C1-C4-,
- C6-C10-Aryl, vorzugsweise C6-C8-Aryl, noch bevorzugter C6-Aryl,
- Halogen, ausgewählt aus Br, Cl oder F, insbesondere Cl, sind und/oder
- wobei zwei der Reste R⁵, R⁶, R⁷ und R⁸ Teil eines nicht-aromatischen C5-C10-Ringsystems, vorzugsweise eines nicht-aromatischen C5-C8-Ringsystems, noch bevorzugter eines nicht-aromatischen C5-C7-Ringsystems sind.

8. Verfahren nach einem der vorstehenden Ansprüche, **dadurch gekennzeichnet, dass** die Reste R⁵, R⁶, R⁷ und R⁸ des mindestens einen Alkans gemäß der allgemeinen Formel (II) H, Methyl, Ethyl, n-Propyl, Isopropyl, n-Butyl, Isobutyl und sec-Butyl, insbesondere bevorzugt H, Methyl, Ethyl, n-Propyl, n-Butyl, Phenyl sind.

9. Verfahren nach einem der vorstehenden Ansprüche, **dadurch gekennzeichnet, dass** zwei der Reste R⁵, R⁶, R⁷ und R⁸ einen Cyclohexanring bilden.

10. Verfahren nach einem der vorstehenden Ansprüche, **dadurch gekennzeichnet, dass** 0,2-3,0 Äquivalente, vorzugsweise 0,3-2,0 Äquivalente, mindestens eines Polychlorids gemäß den allgemeinen Formeln (I) und 1,0-4,0 Äquivalente, vorzugsweise 2,0-3,0 Äquivalente, mindestens eines Alkans gemäß der allgemeinen Formel (II) in das Reaktionsgemisch eingebracht werden.

11. Verfahren nach einem der vorstehenden Ansprüche, **dadurch gekennzeichnet, dass** die Reaktion in einem organischen Lösungsmittel durchgeführt wird, das aus der Gruppe ausgewählt ist, die aus Benzol, Nitrobenzol, 1,2-Dichlorbenzol (oDCB), fluoriertem Benzol und Trifluortoluol besteht.

12. Verfahren nach einem der vorstehenden Ansprüche, **dadurch gekennzeichnet, dass** die Lichtquelle eine LED ist, die Licht mit einer Wellenlänge zwischen 420 nm und 600 nm emittiert, vorzugsweise eine blaue LED, die Licht mit einer Wellenlänge zwischen 420 nm und 470 nm emittiert.

13. Verfahren zur radikalischen Chlorierung von Alkanen, umfassend die Schritte des Bereitstellens einer Mischung aus
mindestens einem Polychlorid gemäß der allgemeinen Formel (I)
[N-R¹ₐ R²_{b} R³_{c} R⁴_{d} ] [Cl-(Cl₂ )ₙ]
wobei die Reste R¹, R², R³und R⁴Alkyl, vorzugsweise C1-C6-Alkyl, oder Aryl, vorzugsweise C6-C10-Aryl, sind,
wobei R¹, R², R³ und R⁴ gleich oder voneinander verschieden sein können,
wobei a, b, c, d unabhängig voneinander 0, 1, 2 oder 3 sind, wobei die Summe aus a+b+c+d stets 4 betragen muss;
wobei n > 0, vorzugsweise n >= 1, noch bevorzugter n = 1-6, am meisten bevorzugt n = 1, 2, 3, 4, und
mindestens einem Alkan gemäß der allgemeinen Formel (II)
CR⁵ₑ R⁶_{f} R⁷_{g} R⁸ₕ
wobei die Reste R⁵ , R⁶ , R⁷ und R⁸ H, Alkyl, Aryl oder Halogen sind oder zwei der Reste R⁵ , R⁶ , R⁷ und R⁸ Teil eines nicht-aromatischen cyclischen Ringsystems sind,
wobei R⁵, R⁶, R⁷ und R⁸ gleich oder voneinander verschieden sein können,
wobei mindestens einer der Reste R⁵, R⁶, R⁷ und R⁸ H ist,
wobei e, f, g, h unabhängig voneinander 0, 1, 2 oder 3, 4 sind, wobei die Summe aus e+f+g+h stets 4 betragen muss; und
Zugabe mindestens eines Radikalinitiators zu den Gemischen und Erhitzen des Gemisches auf eine Temperatur zwischen 80 und 120 °C, vorzugsweise zwischen 90 und 110 °C, noch bevorzugter 100 °C, für 15 bis 60 min, vorzugsweise 20 bis 40 min, noch bevorzugter 25 bis 35 min.

14. Verfahren nach einem der vorstehenden Ansprüche, **dadurch gekennzeichnet, dass** mindestens ein Polychlorid gemäß der allgemeinen Formel (I) in mindestens eine Verbindung gemäß der allgemeinen Formel (III)
[N-R¹ₐ R²_{b} R³_{c} R⁴_{d} ] [Cl-(HCl)ₘ]
umgewandelt wird,
wobei die Reste R¹, R², R³ und R⁴ sowie die Variablen a, b, c, d dieselben sind wie zuvor in mindestens einem der vorstehenden Ansprüche beschrieben, und
wobei m > 0, vorzugsweise m >= 1, noch bevorzugter m = 1-6, am meisten bevorzugt m = 1, 2, 3, 4 ist.

15. Verfahren nach einem der vorstehenden Ansprüche, **dadurch gekennzeichnet, dass** mindestens ein Alkan der allgemeinen Formel (II) in mindestens ein chloriertes Alkan der allgemeinen Formel (IV)
(R⁵ₑ R⁶_{f} R⁷_{g} R⁸ₕ)CClₓ
umgewandelt wird,
wobei mindestens einer der Reste R⁵, R⁶, R⁷und R⁸ wie zuvor in mindestens einem der vorstehenden Ansprüche beschrieben ist,
x 1, 2, 3 oder 4 ist,
wobei R⁵, R⁶, R⁷und R⁸ gleich oder voneinander verschieden sein können, und
wobei e, f, g, h unabhängig voneinander 0, 1, 2 oder 3 sind, wobei die Summe aus e+f+g+h immer 4-x betragen muss (d. h. 4 minus der Anzahl der Chloratome).

## Revendications

1. Procédé de chloration photochimique d'alcanes comprenant les étapes de prévoir un mélange de
au moins un polychlorure selon la formule générale (I)
[N-R¹ₐR²_{b}R³_{c}R⁴_{d}] [Cl-(Cl₂)ₙ]
dans laquelle les fragments R¹, R², R³ et R⁴ représentent alkyle, de préférence alkyle C₁-C₆, ou aryle, de préférence aryle C₆-C₁₀,
dans laquelle R¹, R², R³ et R⁴ peuvent être identiques ou différents les uns des autres,
dans laquelle a, b, c, d sont indépendamment l'un de l'autre 0, 1, 2 ou 3, la somme de a+b+c+d devant toujours être égale à 4 ;
dans laquelle n > 0, de préférence n ≥ 1, plus préférentiellement n = 1-6, encore plus préférentiellement n = 1, 2, 3, 4, et
au moins un alcane selon la formule générale (II)
CR⁵ₑR⁶_{f}R⁷_{g}R⁸ₕ
dans laquelle les fragments R⁵, R⁶, R⁷ et R⁸ représentent H, alkyle, aryle, halogène, ou deux parmi R⁵, R⁶, R⁷ et R⁸ font partie d'un système de cycle cyclique non aromatique,
dans laquelle R⁵, R⁶, R⁷ et R⁸ peuvent être identiques ou différents les uns des autres,
dans laquelle au moins l'un des fragments R⁵, R⁶, R⁷ et R⁸ représente H,
dans laquelle e, f, g, h sont indépendamment l'un de l'autre 0, 1, 2 ou 3, 4, la somme de e+f+g+h devant toujours être égale à 4 ; et
irradier le mélange à une longueur d'onde comprise entre 350 nm et 1000 nm, de préférence dans le domaine du visible entre 400 nm et 800 nm, de préférence entre 420 nm et 780 nm, et encore plus préférentiellement entre 420 nm et 600 nm.

2. Procédé selon la revendication 1, **caractérisé en ce que** les fragments R¹, R², R³ et R⁴ du polychlorure selon la formule générale (I) sont choisis parmi le groupe suivant : méthyle, éthyle, n-propyle, isopropyle, n-butyle, isobutyle et 2-méthylpropyle, de préférence méthyle, éthyle ou n-propyle.

3. Procédé selon l'une quelconque des revendications précédentes, **caractérisé en ce que**, dans le composé ionique de la formule générale (I), a, b, c = 1, 2 ou 3, et d = 0, 1, la somme de a + b + c + d devant toujours être égale à 4.

4. Procédé selon l'une quelconque des revendications précédentes, **caractérisé en ce que** le composé ionique de la formule générale (I) est choisi parmi [NEt₃Me][Cl(Cl₂)ₙ], [NEt₂Me₂][Cl(Cl₂)ₙ], [NEtMe₃][Cl(Cl₂)ₙ], [NBuEt₂Me][Cl(Cl₂)ₙ], [NPr₃Me][Cl(Cl₂)ₙ], [NBu₂Me₂][Cl(Cl₂)ₙ], n étant compris entre 1 et 6, de préférence entre 1 et 4.

5. Procédé selon l'une quelconque des revendications précédentes, **caractérisé en ce que** le composé ionique de la formule générale (I) est choisi parmi [NEt₃Me][Cl(Cl₂)ₙ], [NEt₂Me₂][Cl(Cl₂)ₙ], [NEtMe₃][Cl(Cl₂)ₙ], n étant compris entre 1 et 6, de préférence entre 1 et 4.

6. Procédé selon l'une quelconque des revendications précédentes, **caractérisé en ce que** le composé ionique de la formule générale (I) contient (à l'état chargé) au moins 0,1 g de Cl₂ par g de composé ionique, de préférence au moins 0,2 g de Cl₂ par g de composé ionique, plus préférentiellement au moins 0,3 g de Cl₂ par g de composé ionique, encore plus préférentiellement au moins 0,45 g de Cl₂ par g de composé ionique.

7. Procédé selon l'une quelconque des revendications précédentes, **caractérisé en ce que** les fragments R⁵, R⁶, R⁷ et R⁸ de l'au moins un alcane selon la formule générale (II) sont
- H, et/ou
- alkyle C₁-C₁₀, de préférence alkyle C₁-C₆, plus préférentiellement alkyle C₁-C₄,
- aryle C₆-C₁₀, de préférence aryle C₆-C₈, plus préférentiellement aryle C₆,
- halogène choisi parmi Br, Cl ou F, en particulier Cl, et/ou
- deux parmi R⁵, R⁶, R⁷ et R⁸ faisant partie d'un système de cycle cyclique non aromatique en C₅ à C₁₀, de préférence d'un système de cycle cyclique non aromatique en C₅ à C₈, et plus préférentiellement d'un système de cycle cyclique non aromatique en C₅ à C₇.

8. Procédé selon l'une quelconque des revendications précédentes, **caractérisé en ce que** les fragments R⁵, R⁶, R⁷ et R⁸ de l'au moins un alcane selon la formule générale (II) représentent H, méthyle, éthyle, n-propyle, iso-propyle, n-butyle, iso-butyle et sec-butyle, en particulier de préférence H, méthyle, éthyle, n-propyle, n-butyle ou phényle.

9. Procédé selon l'une quelconque des revendications précédentes, **caractérisé en ce que** deux des fragments R⁵, R⁶, R⁷ et R⁸ forment un cycle cyclohexane.

10. Procédé selon l'une quelconque des revendications précédentes, **caractérisé en ce que** 0,2 à 3,0 équivalents, de préférence 0,3 à 2,0 équivalents, d'au moins un polychlorure selon la formule générale (I) et 1,0 à 4,0 équivalents, de préférence 2,0 à 3,0 équivalents, d'au moins un alcane selon la formule générale (II) sont prévus dans le mélange réactionnel.

11. Procédé selon l'une quelconque des revendications précédentes, **caractérisé en ce que** la réaction est réalisée dans un solvant organique choisi parmi le groupe comprenant le benzène, le nitrobenzène, le 1,2-dichlorobenzène (oDCB), le benzène fluoré et le trifluorotoluène.

12. Procédé selon l'une quelconque des revendications précédentes, **caractérisé en ce que** la source lumineuse est une LED émettant de la lumière à une longueur d'onde comprise entre 420 nm et 600 nm, de préférence une LED bleue émettant de la lumière à une longueur d'onde comprise entre 420 nm et 470 nm.

13. Procédé de chloration radicalaire d'alcanes, comprenant les étapes de prévoir un mélange de
au moins un polychlorure selon la formule générale (I)
[N-R¹ₐR²_{b}R³_{c}R⁴_{d}] [Cl-(Cl₂)ₙ]
dans laquelle les fragments R¹, R², R³ et R⁴ représentent alkyle, de préférence alkyle C₁-C₆, ou aryle, de préférence aryle C₆-C₁₀,
dans laquelle R¹, R², R³ et R⁴ peuvent être identiques ou différents les uns des autres,
dans laquelle a, b, c, d sont indépendamment l'un de l'autre 0, 1, 2 ou 3, la somme de a+b+c+d devant toujours être égale à 4 ;
dans laquelle n > 0, de préférence n ≥ 1, plus préférentiellement n = 1-6, encore plus préférentiellement n = 1, 2, 3, 4, et
au moins un alcane selon la formule générale (II)
CR⁵ₑR⁶_{f}R⁷_{g}R⁸ₕ
dans laquelle les fragments R⁵, R⁶, R⁷ et R⁸ représentent H, alkyle, aryle, halogène, ou deux parmi R⁵, R⁶, R⁷ et R⁸ font partie d'un système de cycle cyclique non aromatique,
dans laquelle R⁵, R⁶, R⁷ et R⁸ peuvent être identiques ou différents les uns des autres,
dans laquelle au moins l'un des fragments R⁵, R⁶, R⁷ et R⁸ représente H,
dans laquelle e, f, g, h sont indépendamment l'un de l'autre 0, 1, 2 ou 3, 4, la somme de e+f+g+h devant toujours être égale à 4 ; et
ajouter au moins un initiateur radicalaire aux mélanges et chauffer le mélange à une température comprise entre 80 et 120 °C, de préférence entre 90 et 110 °C, plus préférentiellement à 100 °C, pendant 15 à 60 minutes, de préférence 20 à 40 minutes, plus préférentiellement 25 à 35 minutes.

14. Procédé selon l'une quelconque des revendications précédentes, **caractérisé en ce qu'**au moins un polychlorure selon la formule générale (I) est converti en au moins un composé selon la formule générale (III)
[N-R¹ₐR²_{b}R³_{c}R⁴ₑ] [Cl-(HCl)ₘ]
dans laquelle les fragments R¹, R², R³ et R⁴ et les variables a, b, c, d sont identiques à celles décrites précédemment dans au moins l'une des revendications précédentes, et
dans laquelle m > 0, de préférence m ≥ 1, plus préférentiellement m = 1-6, encore plus préférentiellement m = 1, 2, 3, 4.

15. Procédé selon l'une quelconque des revendications précédentes, **caractérisé en ce qu'**au moins un alcane selon la formule générale (II) est converti en au moins un alcane chloré selon la formule générale (IV)
(R⁵ₑR⁶_{f}R⁷_{g}R⁸ₕ)CClₓ
dans laquelle au moins l'un des fragments R⁵, R⁶, R⁷ et R⁸ est identique à celui décrit précédemment dans au moins l'une des revendications précédentes,
x est 1, 2, 3, ou 4,
dans laquelle R⁵, R⁶, R⁷ et R⁸ peuvent être identiques ou différents les uns des autres, et
dans laquelle e, f, g, h sont indépendamment l'un de l'autre 0, 1, 2 ou 3, la somme de e+f+g+h devant toujours être égale à 4-x (c'est-à-dire 4 moins le nombre d'atomes de chlore).
